# EUROPEAN PATENT SPECIFICATION

(11) **EP 4 522 762 B1**
(45) Date of publication and mention of the grant of the patent: **22.10.2025**
(21) Application number: 23726037.7
(22) Date of filing: 10.05.2023
(51) Int. Cl.: C12Q 1/25, C12Q 1/34, G01N 33/574, A61P 35/02

(54) **ANALYTICAL METHOD AND THERAPEUTICAL AGENT FOR USE IN COMBINATION WITH L-ASPARAGINASE IN TUMOUR THERAPY**
ANALYTISCHES VERFAHREN UND THERAPEUTISCHES MITTEL ZUR VERWENDUNG IN KOMBINATION MIT L-ASPARAGINASE IN DER TUMORTHERAPIE
PROCÉDÉ ANALYTIQUE ET AGENT THÉRAPEUTIQUE À UTILISER EN COMBINAISON AVEC LA L-ASPARAGINASE DANS LE TRAITEMENT DES TUMEURS

(30) Priority: 10.05.2022 EP 22172665
(43) Date of publication of application: 19.03.2025
(73) Proprietor: Medizinische Hochschule Hannover, 30625 Hannover (DE)
(72) Inventor: HINZE, Laura, 30625 Hannover (DE); IBRAHIM, Nurul Khalida, 30625 Hannover (DE)
(74) Representative: Taruttis, Stefan Georg
(86) International application number: PCT/EP2023/062381
(87) International publication number: WO 2023/217840

(56) References cited:
- WO-A1-2021/247009
- WO-A1-2021/247714
- YOSHIMITSU KAZUNORI ET AL: "Superoxide Dismutase Activity in Leukemic Blasts of Children with Acute Leukemia", ACTA PAEDIATRICA, vol. 73, no. 1, 1 January 1984 (1984-01-01), GB, pages 92 - 96, XP055967448, ISSN: 0803-5253, DOI: 10.1111/j.1651-2227.1984.tb09904.x
- ALACHKAR H ET AL: "Expression and polymorphism (rs4880) of mitochondrial superoxide dismutase (SOD2) and asparaginase induced hepatotoxicity in adult patients with acute lymphoblastic leukemia", THE PHARMACOGENOMICS JOURNAL, NATURE PUBLISHING GROUP, GB, vol. 17, no. 3, 29 March 2016 (2016-03-29), pages 274 - 279, XP037650898, ISSN: 1470-269X, [retrieved on 20160329], DOI: 10.1038/TPJ.2016.7
- RASHEDA SULTANA ET AL: "UBR1 promotes protein kinase quality control and sensitizes cells to Hsp90 inhibition", EXPERIMENTAL CELL RESEARCH, ELSEVIER, AMSTERDAM, NL, vol. 318, no. 1, 20 September 2011 (2011-09-20), pages 53 - 60, XP028115730, ISSN: 0014-4827, [retrieved on 20110929], DOI: 10.1016/J.YEXCR.2011.09.010
- IBRAHIM N K ET AL: "S107: SOD2 PROMOTES ACUTE LEUKEMIA ADAPTATION TO AMINO ACID STARVATION THROUGH THE N-DEGRON PATHWAY", HEMASPHERE - 20220601 - LIPPINCOTT WILLIAMS AND WILKINS, 1 June 2022 (2022-06-01), pages 17 - 18, XP055967291, Retrieved from the Internet <URL:https://journals.lww.com/hemasphere/Fulltext/2022/06003/Abstract_Book_for_the_27th_Congress_of_the.1.aspx> [retrieved on 20221003], DOI: 10.1097/01.HS9.0000852292.38263.b8

## Description

The present invention provides an analytical method for use in combination with tumour treatment making use of L-asparaginase as a therapeutical agent, and provides a therapeutical agent for use in combination with the use of L-asparaginase in tumour therapy.

The analytical method of the invention is suitable for predicting the outcome of tumour therapy by administration of L-asparaginase, e.g. for stratifying patients into a group that is predicted to be susceptible to treatment with L-asparaginase, and another group that is predicted as having a poorer response to treatment with L-asparaginase. This stratification of patients allows the therapeutical agent of the invention to be used in combination with L-asparaginase in the treatment of tumour. The preferred tumour to be analysed and for use of the therapeutic agent in combination with L-asparaginase is one of colorectal cancer and leukemia and, especially acute lymphoblastic leukemia (ALL). Generally herein, tumour and cancer are used interchangeably.

### State of the art

Appel et al., Blood 107, 4244-4249 (2006) analysed the effect of administration of L-asparaginase and PEGylated L-asparaginase in treatment of ALL on the expression of asparagin synthetase. The therapeutic effect of administration of L-asparaginase against cancer cells is seen in the depletion of serum asparagine by enzymatic conversion to aspartate.

Place et al., Lancet Oncol, 1677-1690 (2015) analysed the effects of treatment of ALL with native L-asparaginase and PEGylated L-asparaginase.

Hinze et al., Cancer Discov. 10(11) 1690-1705 (2021) describe the analysis of the WNT/β-catenin signaling pathway in colorectal cancer cell lines which originally were resistant to treatment with L-asparaginase. It was found that treatment of these cells by inhibition of GSK3α induced activation of WNT-signaling, which lead to sensitization of the cancer cells to treatment with L-asparaginase.

WO 2021/247714 A1 describes that acetylation of Lys 68 of SOD2 results in change of dismutase activity to peroxidase.

Yoshimitsu et al., Acta Paediatr Scand 73: 92-96 (1984) describes superoxide dismutase levels in acute leukemia and assume that determination of this level may have a prognostic value for children with acute leukemia.

WO 2021/247009 A1 describe cancer treatment depending on levels of sirtuin (SIRT3) protein, levels of Lys 68 acetylation of manganese superoxide dismutase, and levels of hypoxia-inducible factor 2α (HIF2α) using a specific pentaaza macrocyclic compound.

Alachkar et al., The Parmacogenomics Journal 17, 274-279 (2017), describes that in ALL treatment by asparaginase, the CC genotype of rs4880 of the SOD2 gene is associated with higher hepatotoxicity.

Sultana et al., Experimental Cell Research 318, 53-60 (2011) describes that geldanamycin, an inhibitor of HSP90, in mouse embryonic fibroblasts and human breast cancer cells reduces levels of UBR1.

### Object of the invention

It is an object of the invention to provide an analytical method for predicting the efficacy, or outcome, of treatment of cancer, especially of ALL, with L-asparaginase, especially to identify patients having a cancer, e.g. ALL, that can be expected to be resistant against L-asparaginase therapy. A further object is to provide an improved treatment of cancer using administration of L-asparaginase, especially for treatment of cancer that has been analysed and predicted to have resistance against L-asparaginase treatment.

### Description of the invention

The invention achieves the object by the features of the claims, especially by an analytical method for detecting the level of activity of superoxide dismutase (SOD2, e.g. UniProtKB P04179 (SODM_HUMAN) and/or of UBR1 and/or of UBR2 in a sample originating from a patient for determining the sensitivity for, or resistance against, tumour treatment with L-asparaginase. In this embodiment, the invention provides a method for predicting the efficacy of the use of L-asparaginase for use in tumour treatment, e.g. for determination of suitability of use of L-asparaginase in tumour treatment, and respectively for stratifying patients for tumour therapy by L-asparaginase treatment, or for tumour therapy without L-asparaginase treatment, e.g. irradiation therapy and/or chemotherapy only. The sample preferably comprises or consists of cancer cells, e.g. colorectal cancer cells or leukemia cells isolated from a blood sample originating from the patient. Therein, the level of activity of SOD2 can be e.g. the level of SOD2-K68 acetylation, which can be determined by FACS or Western blotting using immunostaining by an antibody specific for acetylated SOD2-K68, or by a colorimetric assay, e.g. by the use of a superoxide dismutase assay kit, or , preferably, the level of activity of SOD2 can be determined by measuring e.g. the abundance of N-degron pathway targets such as LCP1 and/or AFF2 by western blot and/or by assessing mRNA levels by qRT-PCR.

A high level of activity of SOD2 indicates that the cancer cells are not sensitive, e.g. resistant, to treatment with L-asparaginase, predicting a poor effect of L-asparaginase in anti-cancer treatment. In contrast, a low activity level of SOD2 indicates that the cancer cells are sensitive against treatment with L-asparaginase, predicting effectivity of treatment of cancer cells by L-asparaginase. Accordingly, preferably only patients with a low level of activity of SOD2 in cancer cells are selected for therapy that comprises L-asparaginase for use in treatment. Patients with a high activity of SOD2 in cancer cells are selected for therapy that comprises other pharmaceuticals than L-asparaginase for use in treatment.

A low activity level of SOD2 preferably is an activity of SOD2 as found in cancer cells that are sensitive to treatment with L-asparaginase, e.g. an activity below 50%, below 40%, preferably below 35% or below 30% of the activity of SOD2 in cancer cells that are resistant to L-asparaginase.

Further, the invention provides an inhibitor of SOD2 and/or an inhibitor of UBR2 and/or of UBR1 in combination with L-asparaginase for use in the treatment of a tumour, optionally in combination with a chemotherapeutic agent.

The invention shows that an inhibitor of SOD2 and, separately an inhibitor of UBR2 increases the sensitivity of cancer cells for L-asparaginase. Accordingly, an inhibitor for SOD2 and/or an inhibitor for UBR2 and/or UBR1 in combination with L-asparaginase is suitable for use in the treatment of a tumour.

As UBR1 (E3 ubiquitin-protein ligase 1, UniProt KB: Q8IWV7 (UBR1_HUMAN)) and UBR2 (E3 ubiquitin-protein ligase 2, UniProt KB: Q8IWV8 (UBR2_HUMAN)) are sequence homologues (sequelogs), the increased sensitivity of cancer cells in the presence of an inhibitor of UBR2 could also be recapitulated with an inhibitor of UBR1, which increases sensitivity of the cancer cells for treatment by L-asparaginase. Accordingly, the invention also provides an inhibitor of UBR1 and/or an inhibitor of UBR2 for use in the treatment of cancer, especially during treatment of cancer comprising administration of asparaginase. Preferably, an inhibitor of UBR1 and/or an inhibitor of UBR2 is a binding agent directed against the N-domain of UBR1 and/or against the N-domain of UBR2.

Currently, it is assumed that sensitization of cancer cells to treatment with L-asparaginase by each of SOD2 and/or UBR2 and/or its close relative UBR1 is independent of reactive oxygen species, of cell cycle changes, of alterations of mTOR signaling, and of glutamine anaplerosis. The mechanism that causes sensitization of cancer cells to treatment with L-asparaginase by each of SOD2 and/or UBR2 and UBR1 is currently assumed to be a regulation of protein degeneration by the N-degron pathway, also referred to as N-end rule pathway. Inhibition of SOD2 and/or UBR2/UBR1 is believed to reduce activity of the protein degradation, which protein degradation may be activated in response to L-asparaginase treatment in order to release amino acids to replenish asparagine intracellularly, leading to resistance against L-asparaginase. The inhibition of SOD2 and/or of one or both of UBR2 and UBR1 in combination with administration of L-asparaginase according to the invention is assumed to hinder the release of amino acids by protein degradation in the N-degron pathway, thus supporting the starvation of cancer cells of asparagine due to L-asparaginase.

In addition, it is currently assumed that SOD2 directly binds to UBR1 and/or UBR2, and that in particular the N-domain of the UBR paralogs is important for binding and mediating SOD2-driven protein degradation. It is believed that the N-domain of UBR proteins can represent the sensor for certain amino acids, including asparagine. Thus, screening for therapeutic compounds that block the N-domain to inhibit binding of SOD2 with UBR paralogs is expected as a versatile approach to counteract the resistance of cancer cells towards asparaginase therapy.

Accordingly, the invention also relates to a method for analysis of sensitivity for, or resistance against, tumour treatment with L-asparaginase in a sample originating from a patient by analysing the activity level of UBR1 and/or of UBR2. The activity level of UBR1 and/or of UBR2 can be analysed e.g. by measuring the abundance of N-degron pathway targets such as LCP1 and/or AFF2 by western blot and/or by assessing mRNA levels by qRT-PCR.

Further, the disclosure relates to a process for identifying inhibitors of UBR1 and/or of UBR2, e.g. by using DNA-encoded libraries or custom-made small molecule compound libraries that are selectively interfering with the activity of UBR1 and/or UBR2, in particular with the N-domain.

The invention is now described by way of examples with reference to the figures that show in
- Fig. 1A activity levels of SOD2 in human leukemia cells that are sensitive or resistant to treatment with L-asparaginase,
- Fig. 1B activity levels of SOD2 in human cancer cells, present as xenografts on mice, which cancer cells are sensitive or resistant to treatment with L-asparaginase,
- Fig. 1C the receiver operating characteristic (ROC) analysis of SOD2 activity as a biomarker of susceptibility of cancer cells to L-asparaginase treatment,
- Fig. 2A viability of CCRF-CEM cancer cells after knock-down of SOD2 in L-asparaginase treatment,
- Fig. 2B viability of Jurkat cancer cells after knock-down of SOD2 in L-asparaginase treatment,
- Fig. 2C viability of MOLT4 cancer cells after knock-down of SOD2 in L-asparaginase treatment,
- Fig. 2D viability of NALM-16 cancer cells after knock-down of SOD2 in L-asparaginase treatment,
- Fig. 2E viability of HCT-15 cancer cells after knock-down of SOD2 in L-asparaginase treatment,
- Fig. 2F viability of SW480 cancer cells after knock-down of SOD2 in L-asparaginase treatment,
- Fig. 3A cancer cell viability in transduced cancer cells in presence of Vincristine,
- Fig. 3B cancer cell viability in transduced cancer cells in presence of Dexamethasone,
- Fig. 3C cancer cell viability in transduced cancer cells in presence of Doxorubicin,
- Fig. 3D cancer cell viability in transduced cancer cells in presence of 6-mercaptopurine,
- Fig. 4A viability of Jurkat cancer cells after knock-down of UBR2 in L-asparaginase treatment, and
- Fig. 4B viability of CCRF-CEM cancer cells after knock-down of UBR2 in L-asparaginase treatment,
- Fig. 5A knockdown efficiency of UBR2 using to independent shRNAs (*** p ≤ 0.001)
- Fig. 5B viability of Jurkat cancer cells upon knockdown of UBR2 in L-asparaginase treatment (**** p ≤ 0.0001)
- Fig. 5C knockdown efficiency of UBR1 using to independent shRNAs (** p ≤ 0.01), and
- Fig. 5D viability of Jurkat cancer cells upon knockdown of UBR1 in L-asparaginase treatment (**** p ≤ 0.0001).
- Fig. 6A Co-immunoprecipitation of SOD2 and UBR2 indicating a direct binding of the proteins
- Fig. 6B rescue of viability in Jurkat cancer cells, with an inhibitor of UBR1 upon expression of the N-domain of UBR1 or UBR2, representing the fundamental role of this domain in mediating asparaginase response (**** p ≤ 0.0001, *** p ≤ 0.001, ** p ≤ 0.01, * p < 0.05, n.s. p ≥ 0.05),
- Fig. 6C rescue of viability in Jurkat cancer cells with an inhibitor of UBR2 upon expression of the N-domain of UBR1 or UBR2, representing the fundamental role of this domain in mediating asparaginase response (**** p ≤ 0.0001, *** p ≤ 0.001, ** p ≤ 0.01, * p < 0.05, n.s. p ≥ 0.05),
- Fig 6D expression levels of the N-degron targets AFF2 and LCP1 as mesurements of the acitivty level of SOD2 and/or UBR1 and/or UBR2
- Fig. 6E a workflow for compound screening to inhibit the interaction of SOD2 with UBR2 and/or UBR1, particularly to inhibit the N-domain of UBR1 and/or UBR2. Compounds that are selectively binding the N-domain will be selected for further validation. The inset represents the different described domains of the UBR sequelogs, and schematically shows the interaction of SOD2 with the N-domain.

Generally, statistical significance P was assessed by a two-sided Student's t-test with Welch adjustment, or by a one-way ANOVA with Dunnett's adjustment for multiple comparisons unless otherwise indicated.

### Example 1: Activity level of SOD2 in cancer cells as indicator of sensitivity for L-asparaginase treatment, and inhibition of SOD2 increases sensitivity in L-asparaginase treatment

As representatives of cancer cells, leukemia cell lines (CCRF-CEM, Jurkat, MOLT4, DND41, Loucy, KOPTK1) were used. The cell lines were cultivated and treated with 100 U/L L-asparaginase in the medium, or vehicle (PBS) as control with incubation under cell culture conditions for 48 h.

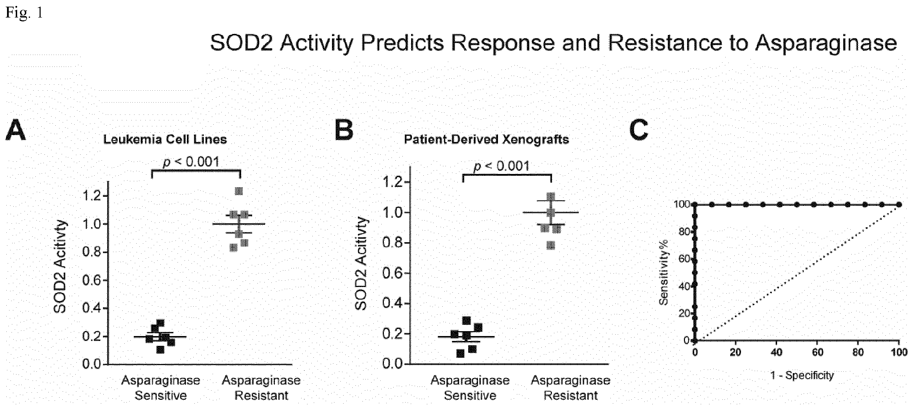

Analysis of the activity level of SOD2 was by Western blotting of total cell proteins, e.g. using immunostaining for SOD2-K68 acetylation with antibody (available from Abcam, catalog number 137037), with quantitative detection of the blot, or by a colorimetric assay, e.g. the Superoxide Dismutase Assay Kit (Cayman 706002), or preferably by assessing the abundance of N-degron targets. The results are depicted in Fig. 1A, showing that low levels of SOD2 activity, e.g. below 40% of the SOD2 activity level found in L-asparaginase resistant cells, correlates with sensitivity to L-asparaginase treatment, whereas high levels of SOD2 activity correlate with resistance against L-asparaginase treatment.

For treatment of PDX cells, ALL clinical specimens collected from children enrolled on ALL-BFM 2000, COALL 0703, COALL 06-97, or AIEOP-BFM ALL 2009 were used, with informed consent and institutional review board approval in accordance with the Declaration of Helsinki. Patient-derived xenografts (PDX) were generated by engrafting viably frozen leukemic cell into immunodeficient mice followed by harvesting and viably freezing, as described in Townsend et al., 2016. PDX cells were thawed, and subsequently cultured in vitro for treatment with vehicle (PBS), or asparaginase (100 U/L) for 48 hrs to assess drug response.

The results show that low activity of SOD2 in the cancer cells correlates with sensitivity to L-asparaginase treatment, whereas a high level of activity of SOD2 correlates with resistance to L-asparaginase.

Fig. 1C for the data shown in Figures 1A and 1B depicts the receiver operating characteristic analysis of SOD2, showing that the activity of SOD2 in cancer cells is a bioindicator suitable for predicting the efficacy of treatment with L-asparaginase.

As a further proof of the correlation of low SOD2 activity in cancer cells with sensitivity for L-asparaginase treatment, resp. of the correlation of high SOD2 activity in cancer cells with resistance against L-asparaginase treatment, T-ALL cells CCRF-CEM, and Jurkat cells, as well as B-ALL cells (NALM-16) and colorectal cancer cells HCT-15, and SW480 were transduced with shRNA that knocks-down SOD2 (shSOD2), or transduced with shRNA that knocks-down luciferase as a control (shLuc), and treated with 0.1 U/L, 1 u/L, 10 U/L, 100 U/L, or 1000 U/L L-asparaginase, followed by incubation under cell culture conditions for 8 days. After the incubation, cell viability was assessed by counting viable cells by Trypan blue exclusion assay. For knock-down of SOD2, shRNA TRCN0000005942 (mature antisense sequence ATAAGGCCTGTTGTTCCTTGC, SEQ ID NO: 1 (shSOD2 #3) or TRCN0000005939 (mature antisense sequence AAAGAGCTTAACATACTCAGC, SEQ ID NO: 2) (shSOD2 #4) were used.

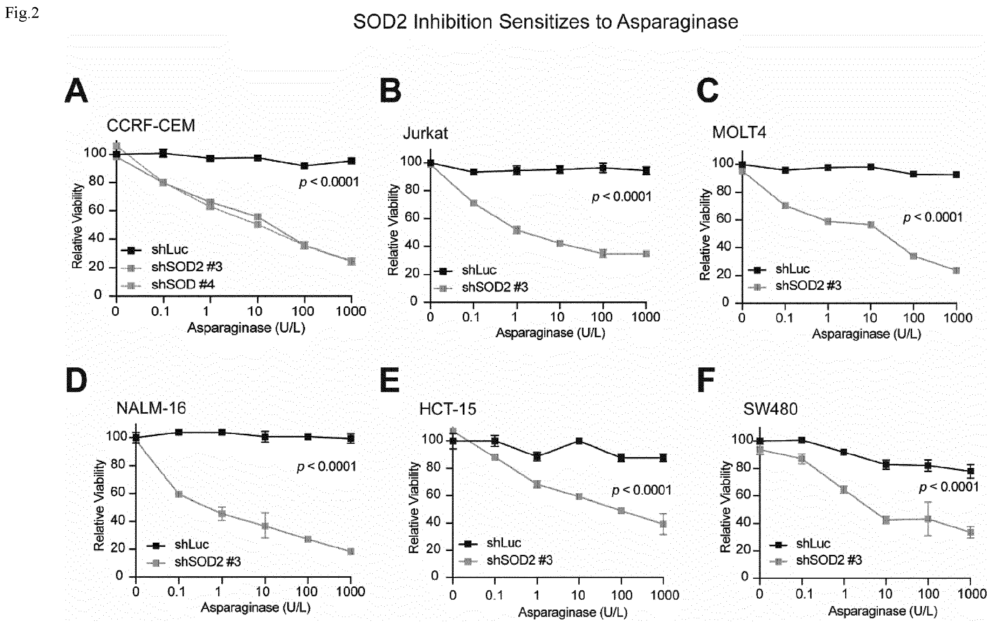

Fig. 2A-F for the different cancer cells depict the anti-cancer activity of L-asparaginase for the cells after shRNA (shSOD) mediated decrease of activity of SOD2, whereas the control-transduced (shLuc) cancer cells continued to be resistant against L-asparaginase treatment. P-values of Fig. 2A-F reflect the comparison of shLuc and shSOD2 at a dose of 100 U/L L-asparaginase and were calculated using a one-way ANOVA with Dunnet's adjustments for multiple comparisons.

These results show that in several types of cancer the sensitivity for L-asparaginase treatment correlates with low activity of SOD2, and that inhibition of activity of SOD2 in cancer increases the sensitivity of the cancer for L-asparaginase. As the results of Example 2 show that the inhibition of SOD2 specifically increases the efficacy of L-asparaginase in the treatment of a tumour, an inhibitor for SOD2 is effective in combination with L-asparaginase alone or in combination with L-asparaginase and a further chemotherapeutic agent for use in treatment of a tumour.

Further, these results show that an inhibitor for SOD2, herein represented by the shRNA that knocks-down SOD2 expression in the cancer cells, is suitable in combination with L-asparaginase for use in the treatment of cancer.

### Example 2: Effect of chemotherapeutic agents for use in treatment of cancer not affected by inhibition of SOD2

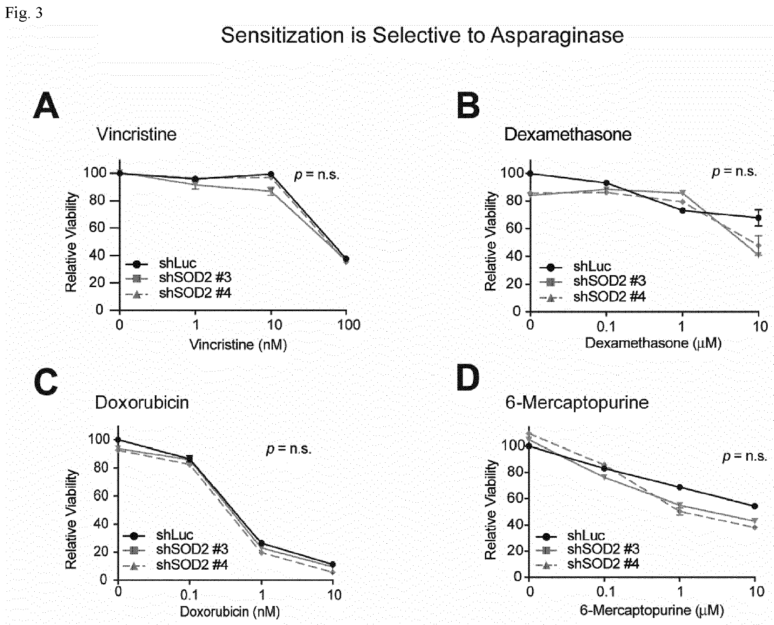

As an example for cancer cells, T-ALL cells (CCRF-CEM) were used in in vitro experiments with a chemotherapeutic agent for treatment of cancer. The cancer cells were transduced with shRNA (shSOD2 #3 or SOD2 #4) that reduces or knock-down expression of SOD2, and shLuc as comparative control (original SOD2 activity maintained). As the chemotherapeutic agent, Vincristine, Dexamethasone, Doxorubicin, or 6-mercaptopurine were used. Following incubation under cell culture conditions for 8 days, cell viability was analysed by Trypan blue exclusion. In Fig. 3A-D, P-values reflect the comparison of cancer cells transduced by shLuc or shSOD2 at the highest dosage of the chemotherapeutic agent.

The results are depicted in Fig. 3A-D, showing that inhibition of SOD2 as caused by knock-down of SOD2 in cancer cells specifically correlates with their sensitivity for L-asparaginase, whereas sensitivity for these chemotherapeutic agents is essentially not affected by the activity level of SOD2.

### Example 3: L-asparaginase in combination with an inhibitor of UBR2 for use in the treatment of cancer

Cas9-expressing Jurkat cells or T-ALL cells (CCRF-CEM) were treated with sgRNA that specifically knocked-out UBR2 (sgUBR2 #3, GAAACTTGAAATAGTCTAAA, SEQ ID NO: 3) or control sgRNA directed against the safe harbor control locus AAVS1 (sgAAVS1, 5'-AGCGGCTCCAATTCGGAAGT-3', SEQ ID NO: 4), and after knock-out of UBR2 cells were incubated under cell-culture conditions with 100 U/L L-asparaginase (Asp) or PBS as control agent (Vehicle). Cell viability was analysed by Trypan blue exclusion after 48 h of incubation.

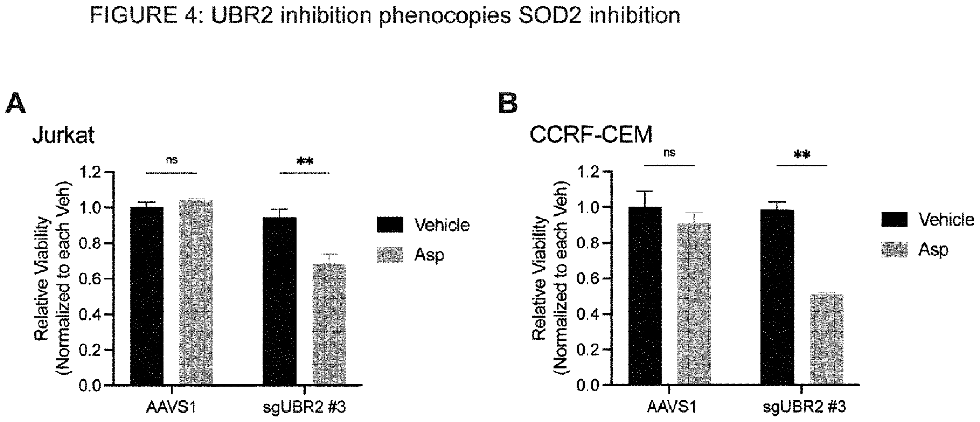

Fig. 4A for Jurkat cells, Fig. 4B for the T-ALL cells depict the cell viability normalized to the cell viability for Vehicle. The results show that the inhibitor for UBR2, represented by the sgRNA (sgUBR2 #3) for knock-down of UBR2, increased the sensitivity of these cells for treatment with L-asparaginase, whereas control inhibitor (AAVS1) essentially had no effect on the sensitivity towards L-asparaginase. P-values reflect the comparison of control inhibitor (AAVS1) and inhibitor of UBR2 (sgUBR2), calculated using a two-way ANOVA with Sidak's adjustments for multiple comparisons.

As an additional experiment, Jurkat cells were treated with two independent shRNAs targeting UBR1 (shUBR1 #1: TRCN0000003423; shUBR1 #4: TRCN0000003424), or UBR2 (shUBR2#2 mature antisense sequence: AAAGGTACCATTCCATTGGT (SEQ ID NO: 5), shUBR2 #3 mature antisense sequence ATATTTCTTGGAGGAAGCAGC (SEQ ID NO: 6), or shLuciferase (TRCN0000072243) as a control. Knockdown efficiency was assessed by Real-time PCR analysis, and upon validation of knockdown, cells were incubated under cell-culture conditions with 100 U/L L-asparaginase (Asp) or PBS as control agent (Vehicle). Cell viability was analysed by Trypan blue exclusion after 4 days of incubation.

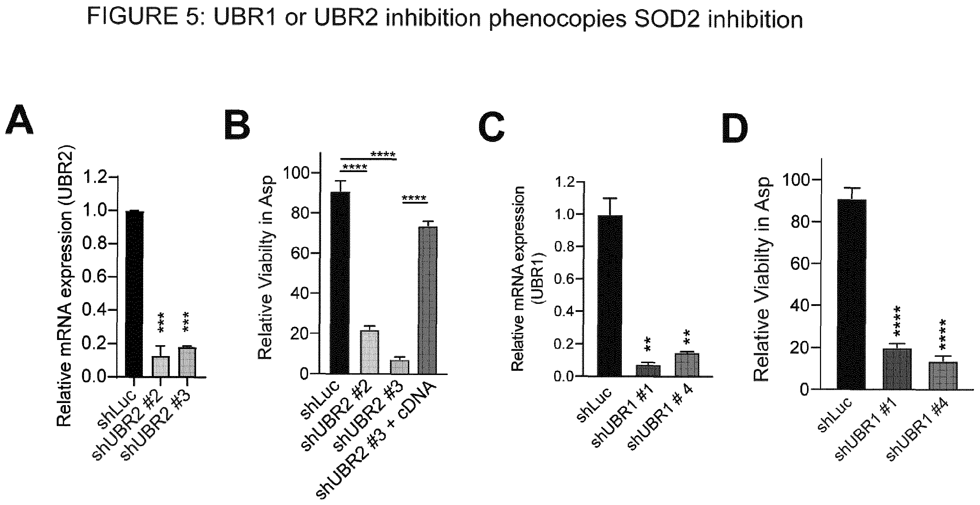

Fig. 5B and 5D depict the cell viability normalized to the cell viability for Vehicle. The results show that the inhibitor for UBR1 or UBR2, represented by shRNAs for knockdown of UBR1 or UBR2, increased the sensitivity of these cells for treatment with L-asparaginase, whereas control inhibitor (shLuc) essentially had no effect on the sensitivity towards L-asparaginase. P-values reflect the comparison of shLuc and inhibitor of UBR1 or UBR2, calculated using a two-way ANOVA with Dunnet's adjustments for multiple comparisons.

### Example 4: Inhibition of the N-domain of UBR1 and/or UBR2 in combination with L-asparaginase in treatment of cancer

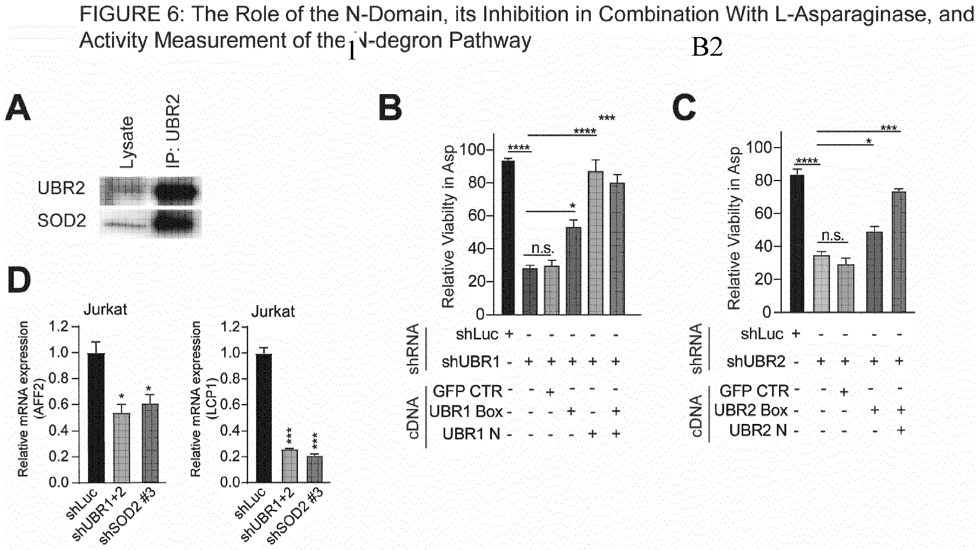

Co-immunoprecipitation (IP: UBR2) revealed a direct binding of SOD2 and UBR2 (Fig. 6A). To identify the domains of UBR sequelogs responsible for mediating asparaginase response, Jurkat cancer cells were first treated with a UBR1 or UBR2 inhibitor, as represented by the use of the shRNAs, followed by overexpression of truncated version of the UBR domains. The UBR N and box domains were defined as follows: UBR1 N domain (amino acid residue: 164-453 of SEQ ID NO: 7), UBR1 box domain (amino acid residues: 1-220 of SEQ ID NO: 7), UBR1 N and box domains (amino acid residue: 1-405 of SEQ ID NO: 7), UBR2 (SEQ ID NO: 8) box domain (amino acid residue: 1-220 of SEQ ID NO: 8), UBR2 N and box domains (amino acid residues: 1-452 of SEQ ID NO: 8). Amino acid numbering was based on UniProt identifiers Q8IWV7 (UBR1) and Q8IWV8 (UBR2).

Fig. 6 B shows cell viability in culture in the presence of L-asparaginase (Asp), without (left column) or with expression of shUBR1 as an inhibitor of UBR1, without (-) or with (+) concurrent expression of cDNA encoding one of GFP as a control (GFP CTR), UBR1 Box, UBR1 N domain (UBR1 N), or both cDNA encoding UBR1 Box and UBR1 N domain. Fig. 6 C shows shows cell viability in culture in the presence of L-asparaginase (Asp), without (left column) or with expression of shUBR2 as an inhibitor of UBR2, without (-) or with (+) concurrent expression of cDNA encoding one of GFP as a control (GFP CTR), UBR2 Box, UBR2 N domain (UBR2 N), or both cDNA encoding UBR2 Box and UBR2 N domain. The N-domain of each of UBR1 and UBR2 was able to rescue asparaginase sensitization, indicating its profound role in mediating response towards this drug. Inhibition of one of UBR1 and UBR2 resulted in increased sensitivity against L-asparaginase, indicated as lower viablility when inhibiting UBR1 or UBR2 by exemplary inhibitors shUBR1 or shUBR2. Cell viability was normalized to shLuc-transduced cells, significance was assessed by a one-way ANOVA with Dunnett's adjustment for multiple comparisons.

Fig 6D shows that inhibition of SOD2 or UBR1 and UBR2 leads to a decrease of the N-degron targets AFF2 and LCP1 indicating an inhibition of the N-degron pathway as a measurement of the activity level of SOD2 and/or UBR1 and/or UBR2.

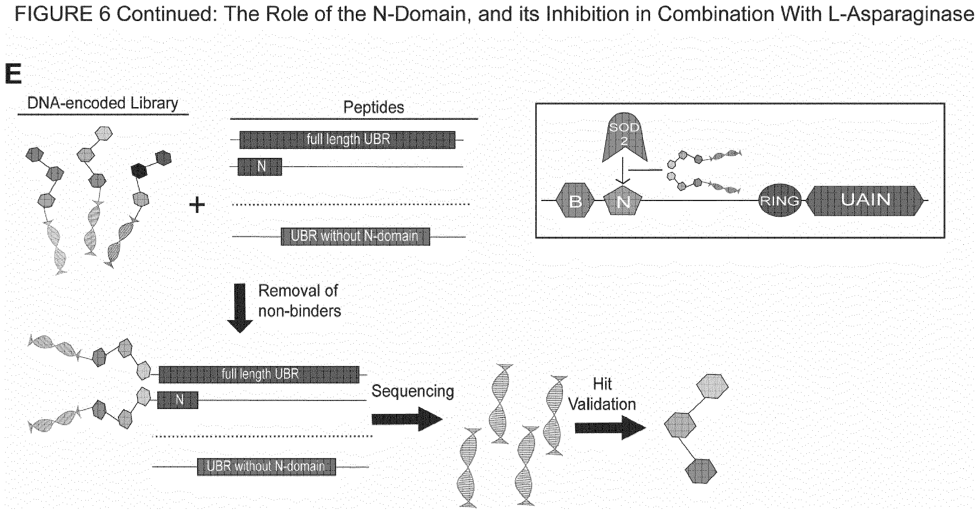

Fig 6E depicts a schematic workflow for screening compounds that can interfere with the binding of SOD2 and UBR1 and/or UBR2, particularly the N-domain. Identification of such a compound is expected to induce asparaginase sensitivity in tumor cells that are dependent on SOD2-driven protein degradation (high activity of SOD2 activity).

## Claims

1. Method for analysis of sensitivity for, or resistance against, tumour treatment with L-asparaginase in a sample originating from a patient, **characterized by** analysing the activity level of UBR1 and/or of UBR2, and/or by analysing the activity of superoxide dismutase 2 (SOD2).

2. Method according to claim 1, wherein a high activity of SOD2 indicates resistance of the tumour against treatment with L-asparaginase, and a low activity of SOD2 indicates susceptibility of the tumour in treatment with L-asparaginase.

3. Method according to one of the preceding claims, wherein a high activity level of UBR1 and/or of UBR2 indicates resistance of the tumour against treatment with L-asparaginase, and a low activity level of UBR1 and/or of UBR2 indicates susceptibility of the tumour in treatment with L-asparaginase.

4. L-asparaginase for use in the treatment of a tumour, **characterized by** a combination of L-asparaginase with an inhibitor of SOD2 and/or an inhibitor of UBR2 and/or an inhibitor of UBR1, for use in the treatment of a tumour.

5. L-asparaginase for use in the treatment of a tumour according to claim 4, **characterized in that** the inhibitor of UBR2 is directed against the N-domain of UBR2, and/or the inhibitor of UBR1 is directed against the N-domain of UBR1.

6. L-asparaginase in combination with an inhibitor of SOD2 and/or an inhibitor of UBR2 and/or of UBR1 for use in the treatment of a tumour according to one of claims 5 to 6, **characterized in that** the tumour was analysed to have a high activity of SOD2.

7. L-asparaginase in combination with an inhibitor of SOD2 and/or an inhibitor of UBR2 and/or of UBR1 for use in the treatment of a tumour according to one of claims 4 to 6, **characterized in that** the tumour was analysed to have a high activity level of UBR1 and/or of UBR2.

8. L-asparaginase in combination with an inhibitor of SOD2 and/or an inhibitor of UBR2 and/or of UBR1 for use in the treatment of a tumour according to one of claims 4 to 7, **characterized in that** the tumour is ALL or colorectal cancer.

## Patentansprüche

1. Verfahren zur Analyse der Empfindlichkeit für oder der Resistenz gegen eine Tumorbehandlung mit L-Asparaginase in einer von einem Patienten stammenden Probe, **gekennzeichnet durch** Analyse des Aktivitätsniveaus von UBR1 und/oder von UBR2 und/oder durch Analyse der Aktivität von Superoxiddismutase 2 (SOD2).

2. Verfahren nach Anspruch 1, wobei eine hohe Aktivität von SOD2 eine Resistenz des Tumors gegen die Behandlung mit L-Asparaginase anzeigt und eine niedrige Aktivität von SOD2 eine Empfindlichkeit des Tumors gegenüber der Behandlung mit L-Asparaginase anzeigt.

3. Verfahren nach einem der vorhergehenden Ansprüche, wobei ein hohes Aktivitätsniveau von UBR1 und/oder von UBR2 eine Resistenz des Tumors gegen die Behandlung mit L-Asparaginase anzeigt und ein niedriges Aktivitätsniveau von UBR1 und/oder von UBR2 eine Anfälligkeit des Tumors für die Behandlung mit L-Asparaginase anzeigt.

4. L-Asparaginase zur Verwendung bei der Behandlung eines Tumors, **gekennzeichnet durch** eine Kombination von L-Asparaginase mit einem Inhibitor von SOD2 und/oder einem Inhibitor von UBR2 und/oder einem Inhibitor von UBR1, zur Verwendung bei der Behandlung eines Tumors.

5. L-Asparaginase zur Verwendung bei der Behandlung eines Tumors nach Anspruch 4, **dadurch gekennzeichnet, dass** der Inhibitor von UBR2 gegen die N-Domäne von UBR2 gerichtet ist und/oder der Inhibitor von UBR1 gegen die N-Domäne von UBR1 gerichtet ist.

6. L-Asparaginase in Kombination mit einem SOD2-Inhibitor und/oder einem UBR2-Inhibitor und/oder einem UBR1-Inhibitor zur Verwendung bei der Behandlung eines Tumors nach einem der Ansprüche 5 bis 6, **dadurch gekennzeichnet, dass** der Tumor so analysiert wurde, dass er eine hohe Aktivität von SOD2 aufweist.

7. L-Asparaginase in Kombination mit einem SOD2-Inhibitor und/oder einem UBR2-Inhibitor und/oder einem UBR1-Inhibitor zur Verwendung bei der Behandlung eines Tumors nach einem der Ansprüche 4 bis 6, **dadurch gekennzeichnet, dass** der Tumor so analysiert wurde, dass er ein hohes Aktivitätsniveau von UBR1 und/oder UBR2 aufweist.

8. L-Asparaginase in Kombination mit einem SOD2-Inhibitor und/oder einem UBR2-Inhibitor und/oder einem UBR1-Inhibitor zur Verwendung bei der Behandlung eines Tumors nach einem der Ansprüche 4 bis 7, **dadurch gekennzeichnet, dass** es sich bei dem Tumor um ALL oder kolorektalen Krebs handelt.

## Revendications

1. Procédé d'analyse de la sensibilité ou de la résistance au traitement tumoral par la L-asparaginase dans un échantillon provenant d'un patient, **caractérisé par** l'analyse du niveau d'activité de l'UBR1 et/ou de l'UBR2, et/ou par l'analyse de l'activité de la superoxyde dismutase 2 (SOD2).

2. Procédé selon la revendication 1, dans lequel une activité élevée de SOD2 indique une résistance de la tumeur au traitement par la L-asparaginase, et une faible activité de SOD2 indique une sensibilité de la tumeur au traitement par la L-asparaginase.

3. Procédé selon l'une des revendications précédentes, dans lequel un niveau d'activité élevé de l'UBR1 et/ou de l'UBR2 indique une résistance de la tumeur au traitement par la L-asparaginase, et un niveau d'activité faible de l'UBR1 et/ou de l'UBR2 indique une sensibilité de la tumeur au traitement par la L-asparaginase.

4. L-asparaginase destinée à être utilisée dans le traitement d'une tumeur, **caractérisée par** une combinaison de L-asparaginase avec un inhibiteur de SOD2 et/ou un inhibiteur de UBR2 et/ou un inhibiteur de UBR1, destinée à être utilisée dans le traitement d'une tumeur.

5. L-asparaginase destinée à être utilisée dans le traitement d'une tumeur selon la revendication 4, **caractérisée en ce que** l'inhibiteur de l'UBR2 est dirigé contre le domaine N de l'UBR2, et/ou l'inhibiteur de l'UBR1 est dirigé contre le domaine N de l'UBR1.

6. L-asparaginase en association avec un inhibiteur de SOD2 et/ou un inhibiteur de UBR2 et/ou de UBR1 pour une utilisation dans le traitement d'une tumeur selon l'une des revendications 5 à 6, **caractérisée en ce que** la tumeur a été analysée comme ayant une activité élevée de SOD2.

7. L-asparaginase en association avec un inhibiteur de SOD2 et/ou un inhibiteur de UBR2 et/ou de UBR1 pour une utilisation dans le traitement d'une tumeur selon l'une des revendications 4 à 6, **caractérisée en ce que** la tumeur a été analysée comme ayant un niveau d'activité élevé de UBR1 et/ou de UBR2.

8. L-asparaginase en association avec un inhibiteur de SOD2 et/ou un inhibiteur de UBR2 et/ou de UBR1 pour une utilisation dans le traitement d'une tumeur selon l'une des revendications 4 à 7, **caractérisée en ce que** la tumeur est une LAL ou un cancer colorectal.
